# EUROPEAN PATENT APPLICATION

(11) **EP 2 228 040 A1**
(43) Date of publication of application: **15.09.2010**
(21) Application number: 09177172.5
(22) Date of filing: 26.11.2009
(51) Int. Cl.: A61F 2/34, A61F 2/00, A61F 2/30

(54) **Acetabular cup implant for artificial hip joint using shape memory alloy**

(30) Priority: 11.03.2009 KR 20090020877
(71) Applicant: Korea Advanced Institute of Science and Technology, Yuseong-gu, Daejeon-city 305-701 (KR)
(72) Inventor: Yoon, Yong San, 305-701, DAEJEON (KR); Bassam, Masri, VANCOUVER British Columbia (CA)
(74) Representative: Bongiovanni, Simone

(57) **Abstract**

Disclosed is an acetabular cup implant for an artificial hip joint fitted to a bone using a shape memory alloy. The acetabular cup implant for an artificial hip joint comprises an acetabular cup for an artificial hip joint formed of a shape memory alloy having a semi-spherical shape with a hollow section and expanded upon arrival at a body temperature to be fitted to a bone, and a bearing installed in the hollow section of the acetabular cup to receive a femoral head to facilitate rotation of a femur. The acetabular cup implant for an artificial hip joint of the present invention can maximize fitting effect of an acetabular cup to a bone using expansion of a shape memory alloy constituting the acetabular cup for an artificial hip joint, and maximally prevent osteolysis due to intrusion of wear particles through a gap or relative movement between the acetabular cup and the bone and separation of the implant from the bone caused by expansive reproduction thereof, without addition of fixation using screws.

## Description

### BACKGROUND OF THE INVENTION

This application claims priority to Korean Patent Application No. 2009-20877, filed on March 11, 2009, in the Korean Intellectual Property Office, the entire contents of which are hereby incorporated by reference.

### 1. Field of the Invention

The present invention relates to an acetabular cup implant for an artificial hip joint fitted to a bone using a shape memory alloy, and more particularly, to an acetabular cup implant for an artificial hip joint which includes an acetabular cup for an artificial hip joint formed of a shape memory alloy having a semi-spherical shape with a hollow section and expanded upon the body temperature to be fitted to a bone, and a bearing installed in the hollow section of the acetabular cup to receive a femoral head to rotate a femur.

### 2. Discussion of the Related Art

An acetabular cup for an artificial hip joint is the most important element in hip arthroplasty, and plays an important role to determine the lifespan of an artificial hip joint. If there is a gap or relative movement between the acetabular cup and the bone, wear particles may intrude to cause osteolysis or create soft tissues, generating larger movement. In this case, these circumstances are expansively reproduced to rapidly deteriorate the artificial hip joint, thereby separating the implant from the bone.

In order to solve these problems, US Patent No. 6,626,947 discloses an acetabular cup press-fitted to a bone with a gap of about 2mm. That is, the semi-spherical acetabular cup having a radius about 2mm larger than that of a semi-spherical space of the bone is press-fitted into the semi-spherical space. However, such press-fitting larger than about 2mm may injure fine tissues of the bone. For this reason, in many cases, separate screws are used to more securely fix the acetabular cup to the bone.

### SUMMARY OF THE INVENTION

Accordingly, it is an object of the present invention to provide an acetabular cup implant for an artificial hip joint capable of maximizing fitting effect of an acetabular cup to a bone by means of expansion of a shape memory alloy constituting the acetabular cup for an artificial hip joint, and maximally preventing osteolysis due to intrusion of wear particles through a gap or relative movement between the acetabular cup and the bone and separation of the implant from the bone caused by expansive reproduction thereof, without addition fixation using screws.

In order to accomplish the above object, the present invention provides an acetabular cup implant for an artificial hip joint fitted to a bone using a shape memory alloy comprising: an acetabular cup having a hollow section, a generally semi-spherical shape, and formed of a shape memory alloy configured to expand upon a body temperature; and a bearing fitted to an inner surface of the hollow section of the semi-spherical acetabular cup and configured to receive a femoral head of a femur to rotate the femur.

The bearing may be inserted into the acetabular cup in a tapered fitting manner.

The bearing may be formed of polymer, metal, or ceramic.

When the bearing is formed of polymer, the exterior of the polymer may be supplemented with a metal band to obtain a friction force of the taper insertion.

In another example embodiment, an acetabular cup of an acetabular cup implant for an artificial hip joint using a shape memory alloy is **characterized in that** the acetabular cup has a hollow section, a generally semi-spherical shape, and is formed of a shape memory alloy configured to expand upon the body temperature.

The acetabular cup of an acetabular cup implant for an artificial hip joint using a shape memory alloy may include a ring-shaped member formed of a shape memory alloy that can be varied in shape by expansion due to heat supply or heat absorption from the exterior, and a body formed of a titanium alloy having no variation in shape.

The shape memory alloy may be nitinol.

The outer surface of the shape memory alloy in contact with the bone may have a bone compatible material.

The outer surface of the ring-shaped memory alloy in contact with the bone may have a bone compatible material.

The bone compatible material may include a titanium bead, a titanium wire, plasma coating or oxidation.

The acetabular cup may further comprise calcium phosphate,hydroxyapatite or bio-ceramic coating applied on the bone compatible material.

The acetabular cup may further include a film configured to minimize contact of the shape memory alloy with body fluids, connect an end of the ring-shaped member to an end of the body to smoothly expand the ring-shaped member, formed of the shape memory alloy or the titanium alloy, and having circumferential wrinkles.

Specific descriptions of other example embodiments will be apparent from the detailed description and the attached drawings.

### BRIEF DESCRIPTION OF THE DRAWINGS

The above and other objects, features and advantages of the present invention will become more apparent to those of ordinary skill in the art by describing in detail example embodiments thereof with reference to the attached drawings, in which:
FIG. 1 is a schematic view showing an acetabular cup implant for an artificial hip joint fitted into a bone;
FIG. 2 is an exploded perspective view of the artificial hip joint;
FIG. 3 is a cross-sectional view of an acetabular cup implant for an artificial hip joint in accordance with an exemplary embodiment of the present invention fitted into a bone; and
FIG. 4 is a cross-sectional view of an acetabular cup implant for an artificial hip joint in accordance with another exemplary embodiment of the present invention fitted into a bone.

### DETAILED DESCRIPTION OF THE INVENTION

Hereinafter, example embodiments of the present invention will be described in detail. However, the present invention is not limited to the embodiments disclosed below, but can be implemented in various forms. The following embodiments are described in order to enable those of ordinary skill in the art to embody and practice the present invention.

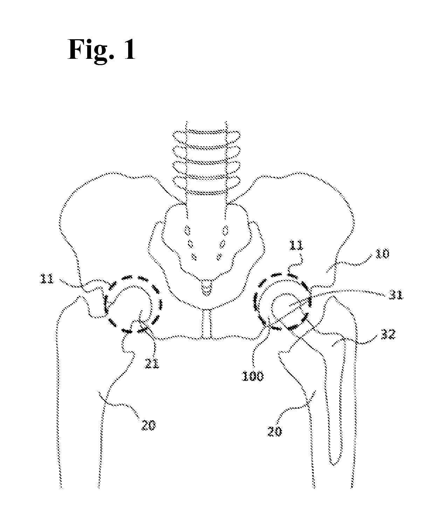

FIG. 1 is a schematic view showing an acetabular cup implant for an artificial hip joint fitted into a left hip joint. In a human body, a femoral head 21 of a femur 20 is inserted into an acetabulum 11 of the pubis 10 to enable free rotation of the femur 20 with regard to the acetabulum 11.

However, bone replacement surgeries are widely operated for patients whose joints are seriously injured to an unrecoverable degree. Reviewing the structure of the artificial hip joint, a ball 31 is inserted into the acetabulum 11 of the pubis 10 so that an acetabular cup 100 can be inserted to be freely rotated. A stem 32 having the ball 31 is inserted into an upper part of the femur 20 and then fixed thereto by bone cement or directly fitted to the stem surface by bone cells themselves.

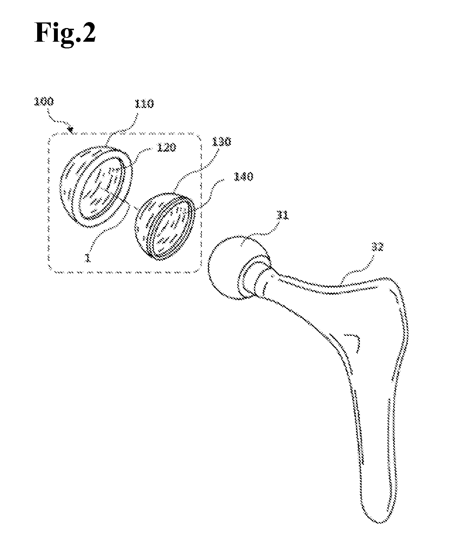

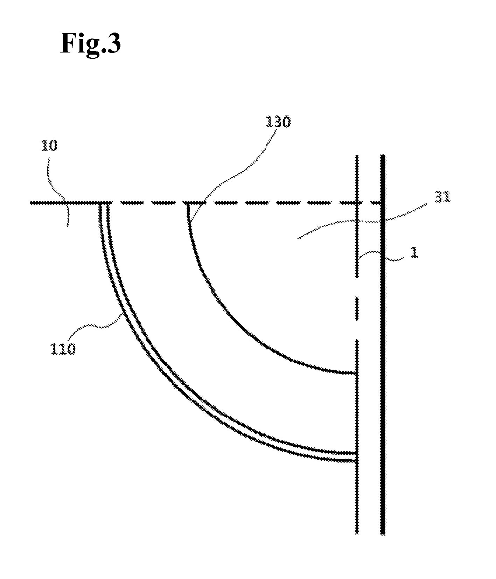

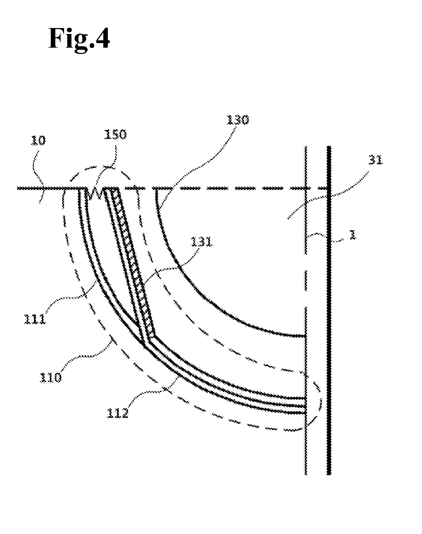

FIG. 2 is an exploded perspective view of the artificial hip joint. The artificial hip joint in accordance with an exemplary embodiment of the present invention includes a stem 32 fixed to the femur at its one end and having a head formed at the other end, a ball 31 coupled to the head, a bearing 130 having a semi-spherical ball receptacle 140 in which the ball 31 is rotatably inserted, and an acetabular cup 110 coupled to the pubis 10, wherein the bearing 130 is inserted into the acetabular cup 110 in a tapered fitting manner. As afore mentioned, the acetabular cup 110 includes a space 120 configured to receive the bearing 130, and is formed of a only shape memory alloy more securely fitted to the pubis 10, or a shape memory alloy and a titanium alloy. The acetabular cup using the shape memory alloy as features of the present invention will be described with reference to FIGS. 3 and 4. The bearing 130 may be formed of various materials such as polymer, metal, ceramic, etc., and fixed to the acetabular cup 110 in the tapered fitting manner as described above.

FIG. 3 is a cross-sectional view of an acetabular cup implant for an artificial hip joint in accordance with an exemplary embodiment of the present invention fitted into a bone (the pubis 10). FIG. 3 shows an embodiment in which the acetabular cup 110 is formed of a shape memory alloy only. When the acetabular cup 110 shown in FIG. 2 is coupled to the bone 10, in the conventional art, the acetabular cup 110 having a radius about 2mm larger than that of the acetabulum 11 of the bone 10 is press-fitted into the acetabulum 11 of the bone 10. Such press-fitting larger than about 2mm may injure fine tissues of the bone 10. For this reason, in many cases, separate screws are used to more securely fix the acetabular cup 110 to the bone 10.

The acetabular cup 110 of the present invention may be formed of a shape memory alloy, for example, nitinol. A primary pressure is generated through slight press-fitting upon an operation, and then, a secondary pressure is generated through expansion of the shape memory alloy due to variation in shape caused by a body heat. The nitinol is shrunk at a temperature lower than the body temperature to be easily inserted into the bone, and then, expanded by variation in shape due to heat supply or heat absorption from the body heat or the exterior, thereby accomplishing the press-fitting pressure. Since the shape memory alloy of the acetabular cup 110 is varied due to the body heat to be expanded and directly fitted to the bone 10, readily accomplishing the press-fitting of 2mm or more, there is no need of additional use of screws in the conventional art. However, since the nitinol is not compatible with the bone 10, the nitinol in contact with the bone 10 may be coated with pure titanium beads, titanium wire meshes, plasma coating, or micro arc oxidation. In addition, calcium phosphate hydroxyapatite or bio-ceramic coating may be added thereon.

The bearing 130 may be formed of various materials such as polymer, metal, ceramic, etc., to be fixed to the acetabular cup 110. In the case of the polymer bearing, the tapered surface may be supplemented with a metal band to obtain a friction force of the tapered fitting.

FIG. 4 is a cross-sectional view of an acetabular cup implant for an artificial hip joint in accordance with another exemplary embodiment of the present invention fitted into a bone. As shown in FIG. 4, the acetabular cup 110 according to this embodiment includes a ring-shaped member 111 formed of a shape memory alloy that can be varied in shape by expansion due to heat supply or heat absorption from the exterior, and a body 112 formed of a titanium alloy having no capability of variation in shape. A primary pressure is generated through slight press-fitting upon operation, and then, a second pressure is generated through expansion of the shape memory alloy due to variation in shape caused by the body heat.

As described above with reference to FIG. 3, the shape memory alloy, for example, nitinol, is shrunk at a temperature lower than the body temperature to be easily inserted into the bone, and then, expanded by variation in shape caused by heat supply or heat absorption from the exterior, thereby accomplishing the press-fitting.

However, since the shape memory alloy of the ring-shaped member 111 is not biologically compatible with the bone 10, in order to minimize a contact with body fluids and the bone 10 and use thereof, a portion requiring no expansion may be replaced with the body 112 formed of a titanium alloy as shown in the drawing. In addition, since the shape memory alloy, i.e., the nitinol is not compatible with the bone 10, the surface of the ring-shaped member 111 in contact with the bone 10 may be coated with pure titanium beads, titanium wire meshes, plasma coating or micro arc oxidation. Further, calcium phosphate hydroxyapatite or bio-ceramic coating may be added thereon.

Meanwhile, in order to smoothly expand the ring-shaped body 111 formed of the shape memory alloy, an end of the ring-shaped body 111 and an end of the body 112 formed of a titanium alloy may be connected to a thin film 150 formed of a shape memory alloy or a titanium alloy and having circumferential wrinkles.

In addition, the bearing 130 may be formed of polymer, metal, ceramic, etc., to be taper-inserted into the acetabular cup 110. In the case of the polymer bearing, the tapered surface may be supplemented with a metal band 131 to obtain a friction force of the tapered fitting.

As can be seen from the foregoing, an acetabular cup implant for an artificial hip joint in accordance with the present invention can maximize fitting effect of an acetabular cup to a bone using expansion of a shape memory alloy constituting the acetabular cup for an artificial hip joint, and maximally prevent osteolysis due to intrusion of wear particles through a gap or relative movement between the acetabular cup and the bone and separation of the implant from the bone caused by expansive reproduction thereof, without addition fixation using screws.

While the invention has been shown and described with reference to certain example embodiments thereof, it will be understood by those skilled in the art that various changes in form and details may be made therein without departing from the spirit and scope of the invention as defined by the appended claims.

## Claims

1. An acetabular cup implant for an artificial hip joint fitted to a bone using a shape memory alloy comprising:
an acetabular cup having a hollow section, a generally semi-spherical shape, and formed of a shape memory alloy configured to expand upon the body temperature; and
a bearing fitted to an inner surface of the hollow section of the semi-spherical acetabular cup and configured to receive a femoral head of a femur to rotate the femur.

2. The implant according to claim 1, wherein the bearing is inserted into the acetabular cup in a tapered fitting manner.

3. The implant according to claim 1, wherein the bearing is formed of polymer, metal, or ceramic.

4. The implant according to claim 3, wherein, when the bearing is formed of polymer, the exterior of the polymer is supplemented with a metal band to obtain a friction force of the taper insertion.

5. An acetabular cup of an acetabular cup implant for an artificial hip joint using a shape memory alloy, **characterized in that** the acetabular cup has a hollow section, a generally semi-spherical shape, and is formed of a shape memory alloy configured to expand upon the body temperature.

6. The acetabular cup according to claim 5, wherein the acetabular cup comprises:
a ring-shaped member formed of a shape memory alloy that can be varied in shape by expansion due to heat supply or heat absorption from the exterior, and
a body formed of a titanium alloy having no variation in shape.

7. The acetabular cup according to claim 5 or 6, wherein the shape memory alloy is nitinol.

8. The acetabular cup according to claim 5, wherein the outer surface of the shape memory alloy contact with the bone has a bone compatible material.

9. The acetabular cup according to claim 6, wherein the outer surface of the ring-shaped memory contact with the bone has a bone compatible material.

10. The acetabular cup according to claim 8 or 9, wherein the bone compatible material includes a titanium bead, a titanium wire, plasma coating or oxidation.

11. The acetabular cup according to claim 8 or 9, further comprising calcium phosphate, hydroxyapatite or bio-ceramic coating applied on the bone compatible material.

12. The acetabular cup according to claim 6, further comprising a film configured to connect an end of the ring-shaped member to an end of the body to smoothly expand the ring-shaped member, formed of the shape memory alloy or the titanium alloy, and having circumferential wrinkles.
